(19) Europäisches Patentamt
European Patent Office
Office européen des brevets

(11) Veröffentlichungsnummer : **0 008 762**
**B1**

(12) **EUROPÄISCHE PATENTSCHRIFT**

(45) Veröffentlichungstag der Patentschrift : **21.10.81**

(51) Int. Cl.³ : **C 07 D239/34**, C 07 D239/70, C 07 D239/90, A 01 N 43/54

(21) Anmeldenummer : **79103155.2**

(22) Anmeldetag : **27.08.79**

(54) **N,N-Dimethyl-O-pyrimidinyl-carbaminsäureester, Verfahren zu ihrer Herstellung und ihre Verwendung als Schädlingsbekämpfungsmittel.**

(30) Priorität : **02.09.78 DE 2838359**

(43) Veröffentlichungstag der Anmeldung : **19.03.80 (Patentblatt 80/06)**

(45) Bekanntmachung des Hinweises auf die Patenterteilung : **21.10.81 Patentblatt 81/42**

(84) Benannte Vertragsstaaten : **BE CH DE FR GB IT NL**

(56) Entgegenhaltungen :
**EP - A - 0 002 200**
**FR - A - 1 060 289**

(73) Patentinhaber : **BAYER AG**
**Zentralbereich Patente, Marken und Lizenzen**
**D-5090 Leverkusen 1, Bayerwerk (DE)**

(72) Erfinder : **Maurer, Fritz, Dr.**
**Roeberstrasse 8**
**D-5600 Wuppertal 1 (DE)**
Erfinder : **Schröder, Rolf, Dr.**
**Pahlkestrasse 17**
**D-5600 Wuppertal 1 (DE)**
Erfinder : **Hammann, Ingeborg, Dr.**
**Belfortstrasse 9**
**D-5000 Köln (DE)**
Erfinder : **Homeyer, Bernhard, Dr.**
**Obere Strasse 28**
**D-5090 Leverkusen 3 (DE)**
Erfinder : **Frohberger, Paul-Ernst, Dr.**
**Willi-Baumeister-Strasse 5**
**D-5090 Leverkusen 1 (DE)**

**EP 0 008 762 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

N,N-Dimethyl-O-pyrimidinyl-carbaminsäureester, Verfahren zu ihrer Herstellung und ihre Verwendung als Schädlingsbekämpfungsmittel

Die Erfindung betrifft neue N,N-Dimethyl-O-pyrimidinyl-carbaminsäureester, mehrere Verfahren zu ihrer Herstellung sowie ihre Verwendung als Schädlingsbekämpfungsmittel, insbesondere als Insektizide und Fungizide.

Es ist bekannt, daß bestimmte N,N-Dialkyl-O-pyrimidinyl-carbaminsäureester, wie z.B. N,N-Dimethyl-O-(2-iso-propyl-6-methyl-pyrimidin(4)yl)- und N,N-Dimethyl-O-(2-methyl-thio-6-methyl-pyrimidin(4)yl)-carbaminsäureester, insektizide Eigenschaften aufweisen (vergleiche FR-PS 1 443 910 und US-PS 2 694 712).

Weiter ist bekannt, daß bestimmte Dithiocarbamate, wie z.B. Zink-äthylen-1,2-bis-(dithiocarbamat), fungizid wirksam sind (vergleiche Phytopathology 33 (1943), 1 113).

Die insektizide bzw. fungizide Wirksamkeit dieser aus dem Stand der Technik bekannten Verbindungen ist jedoch, insbesondere bei niedrigen Wirkstoffkonzentrationen und Aufwandmengen, nicht immer zufriedenstellend.

Es wurden nun neue N,N-Dimethyl-O-pyrimidinyl-carbaminsäureester der Formel I

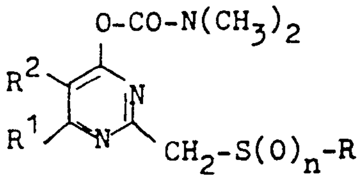
(I)

gefunden, in welcher

R für Alkyl steht,

$R^1$ für Wasserstoff oder Alkyl steht,

$R^2$ für Wasserstoff, Alkyl oder Halogen steht oder $R^1$ und $R^2$ zusammen für Alkandiyl stehen und

n für 1 oder 2 steht.

Man erhält die neuen N,N-Dimethyl-O-pyrimidinyl-carbaminsäureester der Formel (I), wenn man

a) 4-Hydroxy-pyrimidine der Formel (II)

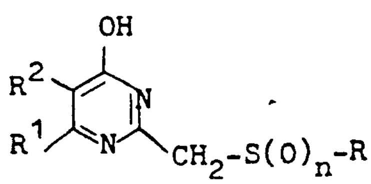
(II)

in welcher

R, $R^1$, $R^2$ und n die oben angegebene Bedeutung haben, mit N,N-Dimethyl-carbaminsäurehalogeniden der Formel (III)

$$Hal-CO-N\ (CH_3)_2 \qquad (III)$$

in welcher

Hal für Chlor oder Brom steht,

gegebenenfalls in Gegenwart eines Säureakzeptors und gegebenenfalls unter Verwendung eines Verdünnungsmittels umsetzt, oder

b) 4-Hydroxy-pyrimidine der Formel (II),

worin R, $R^1$, $R^2$ und n die oben angegebenene Bedeutung haben,

mit Phosgen und anschließend mit Dimethylamin, gegebenenfalls in Gegenwart eines Säureakzeptors und gegebenenfalls unter Verwendung eines Verdünnungsmittels umsetzt.

Verbindungen der Formel (I), in welcher

R, $R^1$ und $R^2$ die oben angegebene Bedeutung haben und n für 1 steht, erhält man auch, inden man

c) N,N-Dimethyl-O-pyrimidin(4)yl-carbaminsäureester der Formel (I), in welcher

R, $R^1$ und $R^2$ die oben angegebene Bedeutung haben und n für Null steht,

mit der äquimolaren Menge Wasserstoffperoxid, gegebenenfalls unter Verwendung eines Verdünnungsmittels umsetzt.

Verbindungen der Formel (I), in welcher

R, $R^1$ und $R^2$ die oben angegebene Bedeutung haben und n für 2 steht, erhält man auch, indem man

d) N,N-Dimethyl-O-pyrimidin(4)yl-carbaminsäureester der Formel (I), in welcher

R, $R^1$ und $R^2$ die oben angegebene Bedeutung haben und n für Null Steht,

mit wenigstens zwei Moläquivalenten m-Chlorperbenzoesäure, gegebenenfalls in Gegenwart eines Verdünnungsmittels umsetzt.

Die neuen N,N-Dimethyl-O-pyrimidinyl-carbaminsäureester der Formel (I) zeichnen sich durch hohe Wirksamkeit als Schädlingsbekämpfungsmittel, insbesondere durch ihre hervorragende insektizide und fungizide Wirkung aus.

Überraschenderweise zeigen die erfindungsgemäßen N,N-Dimethyl-O-pyrimidinyl-carbaminsäureester eine erheblich höhere insektizide Wirkung als die aus dem Stand der Technik bekannten Verbindungen analoger Konstitution und gleicher Wirkungsrichtung und eine wesentlich bessere fungizide Wirkung als das bekannte Zink-äthylen-1,2-bis-(dithiocarbamat).

Gegenstand der Erfindung sind vorzugsweise N,N-Dimethyl-O-pyrimidinyl-carbaminsäureester der Formel (I), in welcher

R für geradkettiges oder verzweigtes Alkyl mit 1 bis 5 Kohlenstoffatomen steht,

$R^1$ für Wasserstoff oder geradkettiges oder verzweigtes Alkyl mit 1 bis 6 Kohlenstoffatomen steht,

$R^2$ für Wasserstoff, geradkettiges oder verzweigtes Alkyl mit 1 bis 6 Kohlenstoffatomen sowie für Chlor oder Brom steht oder

$R^1$ und $R^2$ zusammen für geradkettiges $\alpha$, $\omega$-Alkandiyl mit 3 bis 5 Kohlenstoffatomen stehen und

n für 1 oder 2 steht.

Die Erfindung betrifft insbesondere Verbindungen der Formel (I), in welcher

R für geradkettiges oder verzweigtes Alkyl mit 1 bis 3 Kohlenstoffatomen steht,

$R^1$ für Wasserstoff oder geradkettiges oder verzweigtes Alkyl mit 1 bis 4 Kohlenstoffatomen steht,

$R^2$ für Wasserstoff, geradkettiges oder verzweigtes Alkyl mit 1 bis 4 Kohlenstoffatomen sowie für Chlor oder Brom steht oder

$R^1$ und $R^2$ zusammen für geradkettiges $\alpha$, $\omega$-Alkandiyl mit 3 oder 4 Kohlenstoffatomen stehen und

n für 1 oder 2 steht.

Verwendet man beispielsweise bei Verfahren (a) N,N-Dimethyl-methylcarbaminsäurechlorid, bei Verfahren (b) Phosgen und Dimethylamin und bei beiden Verfahren 2-Äthylsulfonylmethyl-5,6-dimethyl-4-hydroxy-pyrimidin, bei Verfahren (c) N,N-Dimethyl-O-(2-n-propylthiomethyl-5-bromo-6-methyl-pyrimidin (4)yl)-carbaminsäureester und Wasserstoffperoxid und bei Verfahren (d) N,N-Dimethyl-O-(2-methylthiomethyl-5-methyl-6-äthyl-pyrimidin (4)yl)-carbaminsäureester und m-Chlor-perbenzoesäure als Ausgangsstoffe, so können die entsprechenden Reaktionen durch folgende Formelschemata skizziert werden :

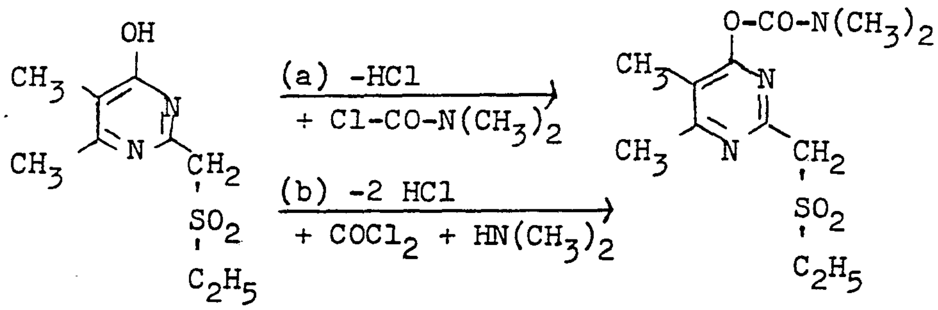


(c)

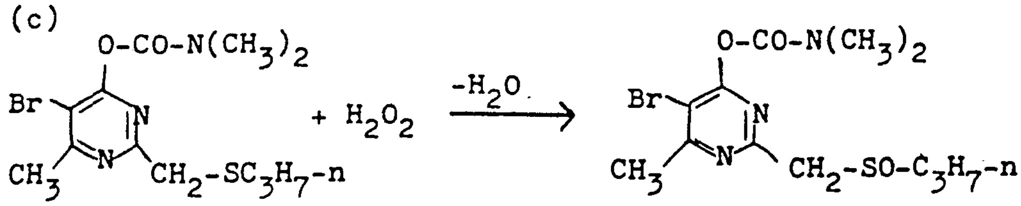


(d)

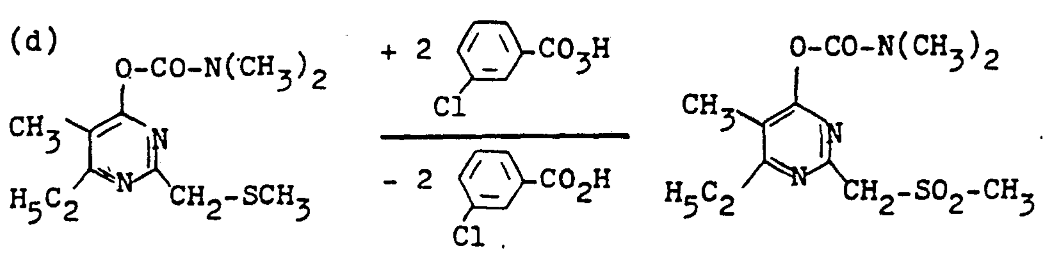

Die bei den Verfahren (a) und (b) als Ausgangsstoffe zu verwendenden 4-Hydroxy-pyrimidine sind durch Formel (II) definiert. Vorzugsweise stehen darin R, $R^1$ und $R^2$ für diejenigen Reste, welche bei der Definition der Reste R, $R^1$ und $R^2$ in Formel (I) als bevorzugt genannt wurden und n steht für 1 oder 2. Die 4-Hydroxy-pyrimidine der Formel (II) erhält man beispielsweise durch Umsetzung der entsprechenden 2-Chlormethyl-4-hydroxy-pyrimidine mit Natriummercaptiden bei Temperaturen zwischen 20 und 100 °C, gegebenenfalls unter Verwendung eines Verdünnungsmittels wie z.B. Acetonitril, Filtration und Abziehen des Verdünnungsmittels und Umsetzung der so erhaltenen 2-Alkylthiomethyl-4-hydroxy-pyrimidine mit einem Oxidationsmittel, wie z.B. Wasserstoffperoxid oder m-Chlorperbenzoesäure, bei Temperaturen zwischen 0 und 40 °C, gegebenenfalls unter Verwendung eines Verdünnungsmittels wie z.B. Essigsäure oder Chloroform.

Als Beispiele für die 4-Hydroxy-pyrimidine der Formel (II) seien genannt :

2-Methylsulfinylmethyl-, 2-Äthylsulfinylmethyl-, 2-n-Propylsulfinylmethyl-, 2-iso-Propylsulfinylmethyl-, 2-Methylsulfonylmethyl-, 2-Äthylsulfonylmethyl-, 2-n-Propylsulfonylmethyl- und 2-iso-Propylsulfonylmethyl-4-hydroxy-pyrimidin,

2-Methylsulfinylmethyl-, 2-Äthylsulfinyl-methyl-, 2-n-Propylsulfinylmethyl-, 2-iso-Propylsulfinylmethyl-, 2-Methylsulfonylmethyl-, 2-Äthylsulfonylmethyl-, 2-n-Propylsulfonylmethyl- und 2-iso-Propylsulfonylmethyl-5-methyl-4-hydroxy-pyrimidin,

2-Methylsulfinylmethyl-, 2-Äthylsulfinylmethyl-, 2-n-Propylsulfinylmethyl-, 2-iso-Propylsulfinylmethyl-, 2-Methylsulfonylmethyl-, 2-Äthylsulfonylmethyl-, 2-n-Propylsulfonylmethyl- und 2-iso-Propylsulfonylmethyl-5-äthyl-4-hydroxy-pyrimidin,

2-Methylsulfinylmethyl-, 2-Äthylsulfinylmethyl-, 2-n-Propylsulfinylmethyl-, 2-iso-Propylsulfinylmethyl-, 2-Methylsulfonylmethyl-, 2-Äthylsulfonylmethyl-, 2-n-Propylsulfonylmethyl- und 2-iso-Propylsulfonylmethyl-5-iso-propyl-4-hydroxy-pyrimidin,

2-Methylsulfinylmethyl-, 2-Äthylsulfinylmethyl-, 2-n-Propylsulfinylmethyl-, 2-iso-Propylsulfinylmethyl-, 2-Methylsulfonylmethyl-, 2-Äthylsulfonylmethyl-, 2-n-Propylsulfonylmethyl- und 2-iso-Propylsulfonylmethyl-6-methyl-4-hydroxy-pyrimidin,

2-Methylsulfinylmethyl-, 2-Äthylsulfinylmethyl-, 2-n-Propylsulfinylmethyl-, 2-iso-Propylsulfinylmethyl-, 2-Methylsulfonylmethyl-, 2-Äthylsulfonylmethyl-, 2-n-Propylsulfonylmethyl- und 2-iso-Propylsulfonylmethyl-5,6-dimethyl-4-hydroxy-pyrimidin,

2-Methylsulfinylmethyl-, 2-Äthylsulfinylmethyl-, 2-n-Propylsulfinylmethyl-, 2-iso-Propylsulfinylmethyl-, 2-Methylsulfonylmethyl-, 2-Äthylsulfonylmethyl-, 2-n-Propylsulfonylmethyl- und 2-iso-Propylsulfonylmethyl-5-äthyl-6-methyl-4-hydroxy-pyrimidin,

2-Methylsulfinylmethyl-, 2-Äthylsulfinylmethyl-, 2-n-Propylsulfinylmethyl-, 2-iso-Propylsulfinylmethyl-, 2-Methylsulfonylmethyl-, 2-Äthylsulfonylmethyl-, 2-n-Propylsulfonylmethyl- und 2-iso-Propylsulfonylmethyl-5-n-propyl-6-methyl-4-hydroxy-pyrimidin,

2-Methylsulfinylmethyl-, 2-Äthylsulfinylmethyl-, 2-n-Propylsulfinylmethyl-, 2-iso-Propylsulfinylmethyl-, 2-Methylsulfonylmethyl-, 2-Äthylsulfonylmethyl-, 2-n-Propylsulfonylmethyl- und 2-iso-Propylsulfonylmethyl-5-iso-propyl-6-methyl-4-hydroxy-pyrimidin,

2-Methylsulfinylmethyl-, 2-Äthylsulfinylmethyl-, 2-n-Propylsulfinylmethyl-, 2-iso-Propylsulfinylmethyl-, 2-Methylsulfonylmethyl-, 2-Äthylsulfonylmethyl-, 2-n-Propylsulfonylmethyl- und 2-iso-Propylsulfonylmethyl-5-n-butyl-6-methyl-4-hydroxy-pyrimidin,

2-Methylsulfinylmethyl-, 2-Äthylsulfinylmethyl-, 2-n-Propylsulfinylmethyl-, 2-iso-Propylsulfinylmethyl-, 2-Methylsulfonylmethyl-, 2-Äthylsulfonylmethyl-, 2-n-Propylsulfonylmethyl- und 2-iso-Propylsulfonylmethyl-5-methyl-6-tert.-butyl-4-hydroxy-pyrimidin,

2-Methylsulfinylmethyl-, 2-Äthylsulfinylmethyl-, 2-n-Propylsulfinylmethyl-, 2-iso-Propylsulfinylmethyl-, 2-Methylsulfonylmethyl-, 2-Äthylsulfonylmethyl-, 2-n-Propylsulfonylmethyl- und 2-iso-Propylsulfonylmethyl-5-chloro-6-methyl-4-hydroxy-pyrimidin,

2-Methylsulfinylmethyl-, 2-Äthylsulfinylmethyl-, 2-n-Propylsulfinylmethyl-, 2-iso-Propylsulfinylmethyl-, 2-Methylsulfonylmethyl-, 2-Äthylsulfonylmethyl-, 2-n-Propylsulfonylmethyl- und 2-iso-Propylsulfonylmethyl-5-bromo-6-methyl-4-hydroxy-pyrimidin,

2-Methylsulfinylmethyl-, 2-Äthylsulfinylmethyl-, 2-n-Propylsulfinylmethyl-, 2-iso-Propylsulfinylmethyl-, 2-Methylsulfonylmethyl-, 2-Äthylsulfonylmethyl-, 2-n-Propylsulfonylmethyl- und 2-iso-Propylsulfonylmethyl-5,6-trimethylen-4-hydroxy-pyrimidin,

2-Methylsulfinylmethyl-, 2-Äthylsulfinylmethyl-, 2-n-Propylsulfinylmethyl-, 2-iso-Propylsulfinylmethyl-, 2-Methylsulfonylmethyl-, 2-Äthylsulfonylmethyl-, 2-n-Propylsulfonylmethyl- und 2-iso-Propylsulfonylmethyl-5,6-tetramethylen-4-hydroxy-pyrimidin.

Als Beispiele für die bei Verfahren (a) zu verwendenden Carbaminsäurehalogenide der Formel (III) sei N,N-Dimethyl-carbaminsäurechlorid genannt. Es ist, ebenso wie die bei Verfahren (b) einzusetzenden Reaktionskomponenten Phosgen und Dimethylamin, lange bekannt.

Die bei den Verfahren (c) und (d) als Ausgangsverbindungen zu verwendenden N,N-Dimethyl-O-pyrimidin (4) ylcarbaminsäureester sind durch die Formel (I) mit der Maßgabe, daß n für Null steht, definiert.

Vorzugsweise stehen darin R, $R^1$ und $R^2$ für diejenigen Reste, welche bei der Definition der Reste R, $R^1$ und $R^2$ in Formel (I) mit der Maßgabe, daß n für 1 oder 2 steht, als bevorzugt genannt wurden. Man erhält die Ausgangsverbindungen der Formel (I), in welcher n für Null steht, analog Verfahren (a),

beispielsweise durch Umsetzung der entsprechenden 2-Alkylthiomethyl-4-hydroxypyrimidine der Formel (II), in welcher n für Null steht, mit N,N-Dimethyl-carbaminsäurechlorid bei Temperaturen zwischen 20 und 100 °C, gegebenenfalls in Gegenwart eines Säureakzeptors wie z.B. Kaliumcarbonat und gegebenenfalls unter Verwendung eines Verdünnungsmittels wie z.B. Acetonitril und Aufarbeitung durch Filtration des Reaktionsgemisches und Abziehen des Verdünnungsmittels.

Als Beispiele für die Ausgangsverbindungen der Formel (I), in welcher n für Null steht, seien genannt: O-(2-Methylthiomethyl-pyrimidin(4)yl)-, O-(2-Äthylthiomethyl-pyrimidin(4)yl)-, O-(2-n-Propylthiomethyl-pyrimidin(4)yl), O-(2-iso-Propylthiomethyl-pyrimidin(4)yl)-, O-(2-Methylthiomethyl-5-methyl-pyrimidin(4)yl)-, O-(2-Äthylthiomethyl-5-methyl-pyrimidin(4)yl)-, O-(2-n-Propylthiomethyl-5-methyl-pyrimidin(4)yl)-, O-(2-iso-Propylthiomethyl-5-methyl-pyrimidin(4)yl)-, O-(2-Methylthiomethyl-5-äthyl-pyrimidin(4)yl)-O-(2-Äthylthiomethyl-5-äthyl-pyrimidin(4)yl)-, O-(2-n-Propylthiomethyl-5-äthyl-pyrimidin(4)yl)-, O-(2-iso-Propylthiomethyl-5-äthyl-pyrimidin(4)yl)-, O-(2-Methylthiomethyl-5-iso-propyl-pyrimidin(4)yl)-, O-(2-Äthylthiomethyl-5-iso-propyl-pyrimidin(4)yl)-, O-(2-n-Propylthiomethyl-5-iso-propyl-pyrimidin(4)yl)-, O-(2-iso-Propylthiomethyl-5-iso-propyl-pyrimidin(4)yl)-, O-(2-Methylthiomethyl-6-methyl-pyrimidin(4)yl)-, O-(2-Äthylthiomethyl-6-methyl-pyrimidin(4)yl)-, O-(2-n-Propylthiomethyl-6-methyl-pyrimidin(4)yl)-, O-(2-iso-Propylthiomethyl-6-methyl-pyrimidin(4)yl)-, O-(2-Methylthiomethyl-5,6-dimethyl-pyrimidin(4)yl)-, O-(2-Äthylthiomethyl-5,6-dimethyl-pyrimidin(4)yl)-, O-(2-n-Propylthiomethyl-5,6-dimethyl-pyrimidin(4)yl)-, O-(2-iso-Propylthiomethyl-5,6-dimethyl-pyrimidin(4)yl)-, O-(2-Methylthiomethyl-5-äthyl-6-methyl-pyrimidin(4)yl)-, O-(2-Äthylthiomethyl-5-äthyl-6-methyl-pyrimidin(4)yl)-, O-(2-n-Propylthiomethyl-5-äthyl-6-methyl-pyrimidin(4)yl)-, O-(2-iso-Propylthiomethyl-5-äthyl-6-methyl-pyrimidin-(4)yl-, O-(2-Methylthiomethyl-5-n-propyl-6-methyl-pyrimidin(4)yl)-, O-(2-Äthylthiomethyl-5-n-propyl-6-methyl-pyrimidin(4)yl)-, O-(2-n-Propylthiomethyl-5-n-propyl-6-methyl-pyrimidin-(4)yl-, O-(2-iso-Propylthiomethyl-5-n-propyl-6-methyl-pyrimidin(4)yl)-, O-(2-Methylthiomethyl-5-iso-propyl-6-methyl-pyrimidin(4)yl)-, O-(2-Äthylthiomethyl-5-iso-propyl-6-methyl-pyrimidin(4)yl)-, O-(2-n-Propylthiomethyl-5-iso-propyl-6-methyl-pyrimidin-(4)yl)-, O-(2-iso-Propylthiomethyl-5-iso-propyl-6-methyl-pyrimidin(4)yl)-, O-(2-Methylthiomethyl-5-n-butyl-6-methyl-pyrimidin(4)yl), O-(2-Äthylthiomethyl-5-n-butyl-6-methyl-pyrimidin(4)yl)-, O-(2-n-Propylthiomethyl-5-n-butyl-6-methyl-pyrimidin(4)yl)-, O-(2-Methylthiomethyl-6-tert.-butyl-5-methyl-pyrimidin(4)yl)-, O-(2-Äthylthiomethyl-6-tert.-butyl-5-methyl-pyrimidin(4)yl)-, O-(2-n-Propylthiomethyl-6-tert.-butyl-5-methyl-pyrimidin(4)yl)-, O-(2-iso-Propylthiomethyl-6-tert.-butyl-5-methyl-pyrimidin(4)yl)-, O-(2-Methylthiomethyl-5-chloro-6-methyl-pyrimidin(4)yl)-, O-(2-Äthylthiomethyl-5-chloro-6-methyl-pyrimidin(4)yl)-, O-(2-n-Propylthiomethyl-5-chloro-6-methyl-pyrimidin(4)yl)-, O-(2-iso-Propylthiomethyl-5-chloro-6-methyl-pyrimidin(4)yl)-, O-(2-Methylthiomethyl-5-bromo-6-methyl-pyrimidin(4)yl)-, O-(2-Äthylthiomethyl-5-bromo-6-methyl-pyrimidin(4)yl)-, O-(2-n-Propylthiomethyl-5-bromo-6-methyl-pyrimidin(4)yl)-, O-(2-iso-Propylthiomethyl-5-bromo-6-methyl-pyrimidin(4)yl)-, O-(2-Methylthiomethyl-5,6-trimethylen-pyrimidin(4)yl)-, O-(2-Äthylthiomethyl-5,6-trimethylen-pyrimidin(4)yl)-, O-(2-n-Propylthiomethyl-5,6-trimethylen-pyrimidin(4)yl)-, O-(2-iso-Propylthiomethyl-5,6-trimethylen-pyrimidin(4)yl)-, O-(2-Methylthiomethyl-5,6-tetramethylen-pyrimidin(4)yl)-, O-(2-Äthylthiomethyl-5,6-tetramethylen-pyrimidin(4)yl)-, O-(2-n-Propylthiomethyl-5,6-tetramethylen-pyrimidin(4)yl)- und O-(2-iso-Propylthiomethyl-5,6-tetramethylen-pyrimidin(4)yl)-N,N-dimethylcarbaminsäureester.

Die bei Verfahren (c) bsw. (d) zu verwendenden Oxidationsmittel Wasserstoffperoxid bzw. m-Chlorperbenzoesäure sind bekannte Verbindungen.

Die Verfahren (a) bis (d) zur Herstellung der neuen N,N-Dimethyl-O-pyrimidinyl-carbaminsäureester werden im allgemeinen unter Verwendung von Verdünnungsmitteln durchgeführt. Als soche kommen parktisch alle inerten organischen Lösungsmittel in Frage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Äther wie Diäthyl- und Dibutyläther, Tetrahydrofuran und Dioxan, Ketone wie aceton, Methyläthyl-, Methylisopropyl- und Methylisobutylketon sowie Nitrile wie Acetonitril und Propionitril.

Verfahren (c) kann vorteilhaft unter Verwendung aliphatischer Carbonsäuren, wie z.B. Ameisensäure, Essigsäure oder Pro ionsäure, als Verdünnungsmittel durchgeführt werden.

Verfahren (a) und (b) werden im allgemeinen unter Verwendung von Säureakzeptoren durchgeführt. Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -äthylat, ferner aliphatische, aromatische oder heterocyclische Amine, beispielsweise Triäthylamin, Trimethylamin, Dimethylanilin, Dimethylbenzylamin und Pyridin.

Die erfindungsgemäßen Verfahren werden im allgemeinen bei Temperaturen zwischen 0 und 150 °C durchgeführt.

Bevorzugt wird für Verfahren (a) der Temperaturbereich zwischen 20 und 100 °C, für Verfahren (b), (c) und (d) der Bereich zwischen 0 und 50 °C. Die Umsetzungen werden im allgemeinen bei Normaldruck durchgeführt.

Zur Durchführung von Verfahren (a) bzw. (b) werden auf 1 Mol 4-Hydroxy-pyrimidin der Formel (II) zwischen 1,0 und 1,3, vorzugsweise zwischen 1,0 und 1,15 Mol N,N-Dimethylcarbaminsäurechlorid bzw. Phosgen und Dimethylamin eingesetzt. Die Umsetzung wird im allgemeinen in einem Verdünnungsmittel in Gegenwart eines Säureakzeptors durchgeführt. Nach Ablauf der Reaktion wird filtriert und das Lösungsmittel im Vakuum abdestilliert.

Bei Verfahren (c) werden die Reaktionskomponenten bevorzugt in äquimolaren Mengen eingesetzt. Bei Verwendung von Verdünnungsmitteln, die mit Wasser mischbar sind, werden diese nach Reaktionsende in Vakuum abdestilliert. Der Rückstand wird dann in einem mit Wasser nicht mischbaren Lösungsmittel, wie z.B. Methylenchlorid, gelöst und nach üblichen Methoden, z.B. durch Waschen, Trocknen, Filtrieren und Abdestillieren des Lösungsmittels aufgearbeitet.

Bei Verfahren (d) wird die als Oxidationsmittel verwendete m-Chlor-perbenzoesäure gewöhnlich im Überschuß eingesetzt, und zwar zwischen 2 und 3 Mol je Mol N,N-Dimethyl-O(2-alkylthiomethyl-pyrimidin(4)yl)-carbaminsäureester. Die Umsetzung wird im allgemeinen in einem mit Wasser nicht mischbaren Lösungsmittel durchgeführt. Nach Ablauf der Reaktion wird neutral gewaschen, getrocknet, filtriert und das Lösungsmittel im Vakuum abdestilliert.

Die neuen Verbindungen fallen in Form von Ölen an, die sich zum Teil nicht unzersetzt destillieren lassen, jedoch durch sogenanntes « Andestillieren », d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Soweit die neuen Verbindungen nach dem Abdestillieren des Lösungsmittels in fester Form anfallen, werden sie durch Umkristallisieren gereinigt. Zur Charakterisierung dient dann der Schmelzpunkt.

Die erfindungsgemäßen N,N-Dimethyl-O-pyrimidinyl-carbaminsäureester zeichnen sich durch hohe insektizide, insbesondere auch wurzelsystemische und fungizide Wirksamkeit aus.

Die Wirkstoffe eignen sich bei guter Pflanzenverträglichkeit und günstiger Warmblütertoxizität zur Bekämpfung von tierischen Schädlingen, insbesondere Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören :

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp.,

Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden,, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisen-oxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

## Beispiel A

Myzus-Test
Lösungsmittel : 3 Gewichtsteile Dimethylformamid
Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden, 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik : 1,3,2,5,8,6,9,4,7,10.

## Beispiel B

Grenzkonzentrations-Test/Wurzelsystemische Wirkung.

Testinsekt : Myzus persicae
Lösungsmittel : 3 Gewichtsteile Aceton
Emulgator : 1 Gewichtsteil Alkylarylpolyglykoläther.

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/1) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik : 10,4,5.

## Beispiel C

Sproßbehandlungs-Test/Getreiderost/protektiv (blattzerstörende Mykose)

Zur Herstellung einer zweckmäßigen Wirkstoffzubereitung nimmt man 0,25 Gew.-Teile Wirkstoff in 25 Gew.-Teilen Dimethylformamid und 0,06 Gew.-Teilen Emulgator Alkylarylpolyglykoläther auf und gibt 975 Gew.-Teile Wasser hinzu. Das Konzentrat verdünnt man mit Wasser auf die gewünschte Endkonzentration in der Spritzbrühe.

Zur Prüfung auf protektive Wirksamkeit inokuliert man einblättrige Weizenjungpflanzen der Sorte Michigan Amber mit einer Uredosporensuspension von Puccinia recondita in 0,1 %igem Wasseragar. Nach Antrocknen der Sporensuspension besprüht man die Weizenpflanzen mit der Wirkstoffzubereitung taufeucht und stellt sie zur Inkubation für 24 Stunden bei etwa 20 °C und einer 100 %igen Luftfeuchtigkeit in ein Gewächshaus.

Nach 10 Tagen Verweilzeit der Pflanzen bei einer Temperatur von 20 °C und einer Luftfeuchtigkeit von 80-90 % wertet man den Besatz der Pflanzen mit Rostpusteln aus. Der Befallsgrad wird in Prozenten des Befalls der unbehandelten Kontrollpflanzen ausgedrückt. Dabei bedeutet 0 % keinen Befall und 100 % den gleichen Befallsgrad wie bei der unbehandelten Kontrolle. Der Wirkstoff ist umso wirksamer, je geringer der Rostbefall ist.

Bei diesem Test zeigen z.B. die folgenden Verbindungen der Herstellungsbeispiele überlegene Wirkung gegenüber dem Stand der Technik : 7

Herstellungsbeispiele

## Beispiel 1

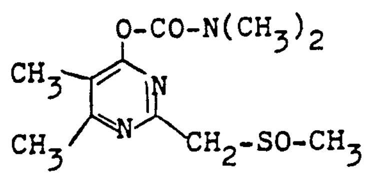

Ein Gemisch aus 20 g (0,1 Mol) 2-Methylsulfinylmethyl-4-hydroxy-5,6-dimethylpyrimidin, 20,7 g (0,15 mol) Kaliumcarbonat, 200 ml Acetonitril und 11,8 g (0,11 Mol) N,N-Dimethylcarbamidsäurechlorid wird 18 Stunden unter Rückfluß gekocht. Nach Abkühlen auf Raumtemperatur filtriert man das Reaktionsgemisch und dampft dann das Filtrat im Vakuum ein. Zurück bleiben 21,3 g (78 % der Theorie) N,N-Dimethyl-O-(2-methylsulfinylmethyl-5,6-dimethyl-pyrimidin(4)yl)-carbaminsäureester in Form beiger Kristalle mit dem Schmelzpunkt 104 °C.

## Beispiel 2

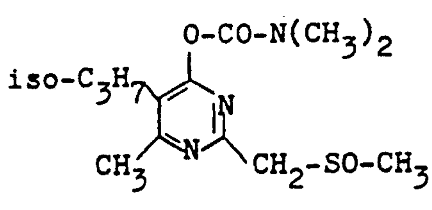

Eine Lösung von 14,2 g (0,05 Mol) N,N-Dimethyl-O-(2-methylthiomethyl-5-iso-propyl-6-methyl-pyrimidin(4)yl)-carbaminsäureester in 50 ml Eisessig wird bei 5-10 °C mit 3,4 g (0,05 Mol) 50 %igem Wasserstoffperoxid versetzt. Man rührt das Gemisch 6 Stunden bei Raumtemperatur nach und destilliert dann das Lösungsmittel im Vakuum ab. Der Rückstand wird in 100 ml Methylenchlorid gelöst und mit einer Lösung von 10 g Kaliumcarbonat in 15 ml Wasser gewaschen. Die organische Phase wird abgetrennt und über Natriumsulfat getrocknet. Dann destilliert man das Lösungsmittel im Vakuum ab. Man erhält so 11 g (74 % der Theorie) N,N-Dimethyl-O-(2-methylsulfinylmethyl-5-iso-propyl-6-methyl-pyrimidin(4)yl)-carbaminsäureester in Form beiger Kristalle mit dem Schmelzpunkt 80 °C.

Beispiel 3

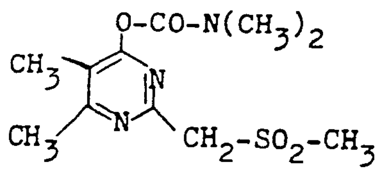

Zu einer Lösung von 17,9 g (0,07 Mol) N,N-Dimethyl-O-(2-methylthiomethyl-5,6-dimethyl-pyrimidin(4)yl-carbaminsäureester in 50 ml Chloroform tropft man bei 5 °C eine Lösung von 31,2 g m-Chlorperbenzoesäure in 250 ml Chloroform. Das Gemisch wird über Nacht bei Raumtemperatur nachgerührt und dann filtriert. Man wäscht das Filtrat mit 10 ml konzentrierten Kaliumcarbonatlösung und trocknet es über Natriumsulfat. Dann wird das Lösungsmittel im Vakuum abgezogen. Zurück bleiben 17 g (85 % der Theorie) N,N-Dimethyl-O-(2-methylsulfonylmethyl-5,6-dimethyl-pyrimidin(4)yl)-carbaminsäureester in Form beiger Kristalle mit dem Schmelzpunkt 122 °C.

Analog einem der Beispiele 1 bis 3 können die folgenden Verbindungen der Formel

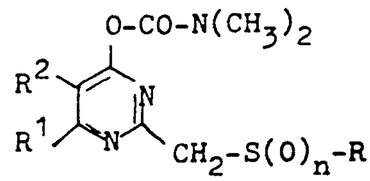

hergestellt werden:

| Beispiel Nr. | R | $R^1$ | $R^2$ | n | Ausbeute (% der Theorie) | Physikal. Daten (Brechungsindex; Schmelzpunkt °C) |
|---|---|---|---|---|---|---|
| 4 | $CH_3$ | $CH_2—CH_2—CH_2$ | | 2 | 72 | 109 |
| 5 | $CH_3$ | $CH_3$ | $C_3H_7$—iso | 2 | 70 | 142 |
| 6 | $CH_3$ | $CH_3$ | $C_4H_9$—n | 2 | 61 | 76 |
| 7 | $CH_3$ | $CH_2—CH_2—CH_2—CH_2$ | | 1 | 84 | 134 |
| 8 | $CH_3$ | $CH_3$ | $C_4H_9$—n | 1 | 96 | $n_D^{20}$ : 1,4557 |
| 9 | $CH_3$ | $CH_2—CH_2—CH_2$ | | 1 | 61 | 115 |
| 10 | $CH_3$ | $CH_2—CH_2—CH_2—CH_2$ | | 2 | 77 | 130 |
| 11 | $C_2H_5$ | $CH_3$ | H | 1 | | |
| 12 | $C_2H_5$ | $CH_3$ | $CH_3$ | 1 | 66 | |
| 13 | $C_2H_5$ | $CH_3$ | H | 2 | | |
| 14 | $C_2H_5$ | $CH_3$ | $CH_3$ | 2 | 40 | |
| 15 | $CH_3$ | $CH_3$ | $C_2H_5$ | 1 | 91 | $n_D^{21}$ : 1,5351 |
| 16 | $CH_3$ | $CH_3$ | $C_2H_5$ | 2 | 66 | 88 |
| 17 | $CH_3$ | $CH_3$ | $C_3H_7$—n | 1 | 94 | $n^{21}$ : 1,5293 |
| 18 | $CH_3$ | $CH_3$ | $C_3H_7$—n | 2 | 83 | 103 |
| 19 | $C_2H_5$ | $CH_2—CH_2—CH_2$ | | 1 | 87 | 97 |
| 20 | $C_2H_5$ | $CH_2—CH_2—CH_2$ | | 2 | 89 | $n_D^{25}$ : 1,5311 |
| 21 | $C_3H_7$—n | $CH_2—CH_2—CH_2$ | | 1 | | |
| 22 | $C_3H_7$—n | $CH_2—CH_2—CH_2$ | | 2 | | |
| 23 | $CH_3$ | H | $CH_3$ | 1 | | |
| 24 | $CH_3$ | H | $CH_3$ | 2 | | |
| 25 | $CH_3$ | H | $C_3H_7$—iso | 1 | | |
| 26 | $CH_3$ | H | $C_3H_7$—iso | 2 | | |

| Beispiel Nr. | R | $R^1$ | $R^2$ | n | Ausbeute (% der Theorie) | Physikal. Daten (Brechungsindex; Schmelzpunkt °C) |
|---|---|---|---|---|---|---|
| 27 | $CH_3$ | H | H | 1 | | |
| 28 | $CH_3$ | H | H | 2 | | |
| 29 | $CH_3$ | $CH_3$ | H | 1 | | |
| 30 | $CH_3$ | $CH_3$ | H | 2 | | |
| 31 | $C_2H_5$ | H | $C_3H_7$—iso | 1 | | |
| 32 | $C_2H_5$ | H | $C_3H_7$—iso | 2 | | |
| 33 | $CH_3$ | H | $C_2H_5$ | 1 | | |
| 34 | $CH_3$ | H | $C_2H_5$ | 2 | | |
| 35 | $CH_3$ | $CH_3$ | Br | 1 | | |
| 36 | $CH_3$ | $CH_3$ | Br | 2 | | |
| 37 | $CH_3$ | $CH_3$ | Cl | 1 | | |
| 38 | $CH_3$ | $CH_3$ | Cl | 2 | | |
| 39 | $CH_3$ | $C_4H_9$—tert. | $CH_3$ | 1 | | |
| 40 | $CH_3$ | $C_4H_9$—tert. | $CH_3$ | 2 | | |

Die Herstellung der Ausgangsverbindungen wird durch folgende Beispiele erläutert:

(a)

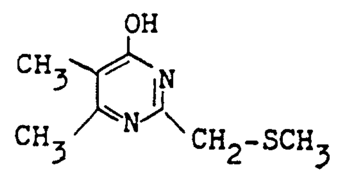

Ein Gemisch aus 129 g (0,75 Mol) 2-Chloromethyl-5,6-dimethyl-4-hydroxy-pyrimidin, 52,5 g (0,75 Mol) Natriummethylmercaptid und 700 ml Acetonitril wird 3 Stunden unter Rückfluß zum Sieden erhitzt. Das heiße Reaktionsgemisch wird filtriert und das Filtrat im Vakuum zur Trockne eingedampft. Man erhält 45,5 g (33 % der Theorie) 2-Methylthiomethyl-5,6-dimethyl-4-hydroxy-pyrimidin in Form eines beigen Pulvers mit dem Schmelzpunkt 142 °C.

(b)

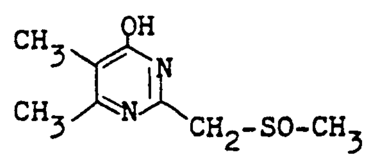

Eine Mischung aus 18,4 g (0,1 Mol) 2-Methylthiomethyl-4-hydroxy-5,6-dimethylpyrimidin in 100 ml Eisessig wird bei 10 °C mit 6,8 g (0,1 Mol) 50 %igem Wasserstoffperoxid versetzt. Man rührt das Gemisch 3 Stunden ohne Kühlung nach, gibt ca. 300 ml Äther zu und saugt das ausgefallene Produkt ab. Man erhält so 17 g (85 % der Theorie) 2-Methylsulfinylmethyl-4-hydroxy-5,6-dimethylpyrimidin in Form eines grauen Pulvers mit dem Schmelzpunkt 112 °C.

(c)

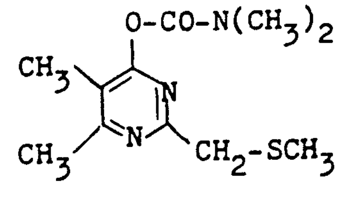

Ein Gemisch aus 18,5 g (0,1 Mol) 2-Methylthiomethyl-5,6-dimethyl-4-hydroxy-pyrimidin, 20,7 g (0,15 Mol) Kaliumcarbonat, 11,8 g (0,11 Mol) N,N-Dimethyl-carbaminsäurechlorid und 200 ml Acetonitril wird 12 Stunden unter Rückfluß zum Sieden erhitzt. Nach Abkühlen auf Raumtemperatur wird das Reaktionsgemisch filtriert und das Filtrat in Vakuum eingedampft. Zurück bleiben 19,1 g (75 % der Theorie) N,N-Dimethyl-O-(2-methylthiomethyl-5,6-dimethyl-pyrimidin(4)yl)-carbaminsäureester in Form eines braunen Öles mit dem Brechungsindex $n_D^{23}$: 1,5491.

**Ansprüche**

1. N,N-Dimethyl-O-pyrimidinyl-carbaminsäureester der Formel (I)

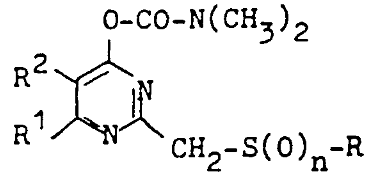

(I)

gefunden, in welcher

R für Alkyl steht,

$R^1$ für Wasserstoff oder Alkyl steht,

$R^2$ für Wasserstoff, Alkyl oder Halogen steht oder $R^1$ und $R^2$ zusammen für Alkandiyl stehen und

n für 1 oder 2 steht.

2. Verfahren zur Herstellung der N,N-Dimethyl-O-pyrimidinyl-carbaminsäureester der Formel (I) gemäß Anspruch 1, dadurch gekennzeichnet, daß man

a) 4-Hydroxy-pyrimidine der Formel (III)

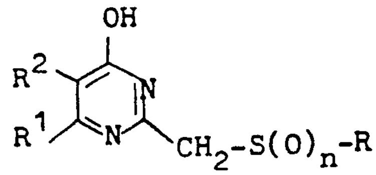

(II)

in welcher

R, $R^1$, $R^2$ und n die oben angegebene Bedeutung haben, mit N,N-Dimethyl-carbaminsäurehalogeniden der Formel (III)

$$Hal-CO-N(CH_3)_2 \qquad (III)$$

in welcher

Hal für Chlor oder Brom steht,

gegebenenfalls in Gegenwart eines Säureakzeptors und gegebenenfalls unter Verwendung eines Verdünnungsmittels umsetzt, oder

b) 4-Hydroxy-pyrimidine der Formel (II),

worin R, $R^1$, $R^2$ und n die oben angegebene Bedeutung haben, mit Phosgen und anschließend mit Dimethylamin, gegebenenfalls in Gegenwart eines Säureakzeptors und gegebenenfalls unter Verwendung eines Verdünnungsmittels umsetzt, oder für den Fall, daß Verbindungen der Formel (I), in welcher R, $R^1$ und $R^2$ die oben angegebene Bedeutung haben und n für 1 steht, hergestellt werden sollen, indem man

c) N,N-Dimethyl-O-pyrimidin(4)yl-carbaminsäureester der Formel (I), in welcher R, $R^1$ und $R^2$ die oben angegebene Bedeutung haben und n für Null steht,

mit der äquimolaren Menge Wasserstoffperoxid, gegebenenfalls unter Verwendung eines Verdünnungsmittels umsetzt, oder für den Fall, daß

Verbindungen der Formel (I), in welcher R, $R^1$ und $R^2$ die oben angegebene Bedeutung haben und n für 2 steht, hergestellt werden sollen, indem man

d) N,N-Dimethyl-O-pyrimidin(4)yl-carbaminsäureester der Formel (I) in welcher R, $R^1$ und $R^2$ die oben angegebene Bedeutung haben und n für Null steht,

mit wenigstens zwei Moläquivalenten m-Chlorbenzoesäure, gegebenenfalls in Gegenwart eines Verdünnungsmittels umsetzt.

3. Insektizide und/oder fungizide Mittel, gekennzeichnet durch einen Gehalt an mindestens einem N,N-Dimethyl-O-pyrimidinyl-carbaminsäureester der Formel (I) gemäß Anspruch 1.

4. Verwendung von N,N-Dimethyl-O-pyrimidinyl-carbaminsäureestern der Formel (I) gemäß Anspruch 1 zur Bekämpfung von Insekten und/oder pflanzenpathogenen Pilzen.

5. Verfahren zur Herstellung insektizider und/oder fungizider nematizider Mittel, dadurch gekennzeichnet, daß man N,N-Dimethyl-O-pyrimidinyl-carbaminsäureester der Formel (I) gemäß Anspruch 1 mit Streckmitteln und/oder oberflächenaktiven Mitteln vermischt.

**Claims**

1. N,N-Dimethyl-carbamic acid O-pyrimidinyl esters of the formula (I)

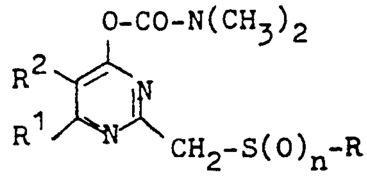

(I)

found, in which

R represents alkyl,
$R^1$ represents hydrogen or alkyl,
$R^2$ reprensents hydrogen, alkyl or halogen, or
$R^1$ and $R^2$ together represent alkanediyl, and
n represents 1 or 2.

2. Process for the preparation of the N,N-dimethylcarbamic acid-O-pyrimidinyl esters of the formula (I), according to Claim 1, characterised in that

a) 4-hydroxy-pyrimidines of the formula (II)

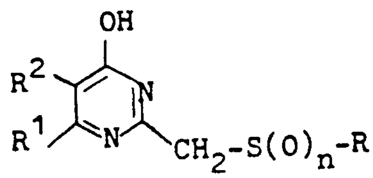

(II)

in which

R, $R^1$, $R^2$ and n have the meaning indicated above, are reacted with N,N-dimethyl-carbamic acid halides of the formula (III)

$$Hal-CO-N(CH_3)_2 \quad (III)$$

in which

Hal represents chlorine or bromine, if appropriate in the presence of an acid acceptor and if appropriate using a diluent, or

b) 4-hydroxy-pyrimidines of the formula (II) wherein R, $R^1$, $R^2$ and n have the meaning indicated above, are reacted with phosgene and the products are then reacted with dimethylamine, if appropriate in the presence of an acid acceptor and if appropriate using a diluant, or, in the case where compounds of the formula (I) in which R, $R^1$ and $R^2$ have the meaning indicated above and n represents 1 are to be prepared, by

c) reacting N,N-dimethyl-carbamic acid-O-pyrimidin-4-yl esters of the formula (I) in which R, $R^1$ and $R^2$ have the meaning indicated above and n represents zero,
with an equimolar amount of hydrogen peroxide, if appropriate using a diluent, or, in the case where compounds of the formula (I) in which R, $R^1$ and $R^2$ have the meaning indicated above and n represents 2, are to be prepared, by

d) reacting N,N-dimethyl-carbamic acid O-pyrimidin-4-yl esters of the formula (I) in which R, $R^1$ and $R^2$ have, the meaning indicated above and n represents zero,
with at least two molar equivalents of m-chlorobenzoic acid, if appropriate in the presence of a diluent.

3. Insecticidal and/or fungicidal agents, characterised in that they contain at least one N,N-dimethyl-carbamic acid O-pyrimidinyl ester of the formula (I) according to Claim 1.

4. Use of N,N-dimethyl-carbamic acid O-pyrimidinyl esters of the formula (I) according to Claim 1 for combating insects and/or phytopathogenic fungi.

5. Process for the preparation of insecticidal and/or fungicidal nematicidal agents, characterised in that N,N-dimethyl-carbamic acid O-pyrimidinyl esters of the formula (I) according to Claim 1 are mixed with extenders and/or surface-active agents.

**Revendications**

1. Esters d'acides N,N-diméthyl-O-pyrimidinyl-carbamiques de formule (I) :

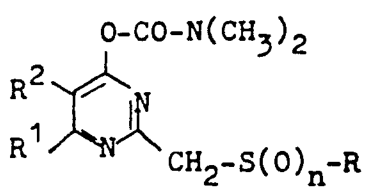

(I)

dans laquelle

R représente un groupe alkyle,

$R^1$ représente un atome d'hydrogène ou un groupe alkyle,

$R^2$ représente un atome d'hydrogène, un groupe alkyle ou un atome d'halogène, ou encore $R^1$ et $R^2$ ensemble, représentent un groupe alcanediyle, et

n est égal à 1 ou 2.

2. Procédé de préparation des esters d'acides N,N-diméthyl-O-pyrimidinyl-carbamiques de formule (I) suivant la revendication 1, caractérisé en ce que :

a) on fait réagir des 4-hydroxy-pyrimidines de formule (II) :

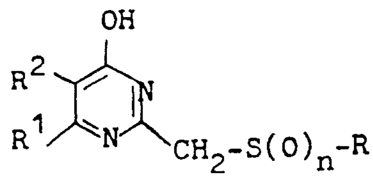

(II)

dans laquelle

R, $R^1$, $R^2$ et n ont les significations indiquées ci-dessus, avec des halogénures d'acides N,N-diméthyl-carbamiques de formule (III) :

$$Hal-CO-N(CH_3)_2 \qquad (III)$$

dans laquelle

Hal représente un atome de chlore ou un atome de brome, éventuellement en présence d'un accepteur d'acide et éventuellement en utilisant un diluant, ou

b) on fait réagir des 4-hydroxy-pyrimidines de formule (II) dans laquelle R, $R^1$, $R^2$ et n ont les significations indiquées ci-dessus, avec du phosgène, puis avec de la diméthylamine, éventuellement en présence d'un accepteur d'acide et éventuellement en utilisant un diluant ou, si l'on veut préparer des composés de formule (I) dans laquelle R, $R^1$ et $R^2$ ont les significations indiquées ci-dessus et n est égal à 1,

c) on fait réagir des esters d'acides N,N-diméthyl-O-pyrimidin(4)yl-carbamiques de formule (I) dans laquelle R, $R^1$ et $R^2$ ont les significations indiquées ci-dessus et n est égal à zéro, avec une quantité équimolaire de peroxyde d'hydrogène, en utilisant éventuellement un diluant ou, si l'on veut préparer des composés de formule (I) dans laquelle R, $R^1$ et $R^2$ ont les significations indiquées ci-dessus et n est égal à zéro,

d) on fait réagir des esters d'acides N,N-diméthyl-O-pyrimidin(4)yl-carbamiques de formule (I) dans laquelle R, $R^1$ et $R^2$ ont les significations indiquées ci-dessus et n est égal à zéro, avec au moins deux équivalents molaires d'acide m-chlorobenzoïque, éventuellement en présence d'un diluant.

3. Agents insecticides et/ou fongicides, caractérisés en ce qu'ils contiennent au moins un ester d'acide N,N-diméthyl-O-pyrimidinyl-carbamique de formule (I) suivant la revendication 1.

4. Utilisation d'esters d'acides N,N-diméthyl-O-pyrimidinyl-carbamiques de formule (I) suivant la revendication 1 en vue de combattre les insectes et/ou les champignons pathogènes des plantes.

5. Procédé de préparation d'agents nématocides, insecticides et/ou fongicides, caractérisé en ce qu'on mélange des esters d'acides N,N-diméthyl-O-pyrimidinyl-carbamiques de formule (I) suivant la revendication 1 avec dees diluants et/ou des agents tensio-actifs.